(19) Europäisches Patentamt
European Patent Office
Office européen des brevets

(11) Publication number: **0 074 777**
**B1**

(12) **EUROPEAN PATENT SPECIFICATION**

(45) Date of publication of patent specification: **14.10.87**

(21) Application number: **82304673.5**

(22) Date of filing: **07.09.82**

(51) Int. Cl.[4]: **C 07 D 519/00,**
C07 D 499/32, A 61 K 31/43//
C07D499/24, C07D499/00,
(C07D519/00, 499:00,
499:00),(C07D519/00, 499:00,
498:00)

(54) **Antibacterial 6-(2-amino-2-(4-acyloxy-phenyl) acetamido) penicillanoyloxymethyl esters.**

(30) Priority: **09.09.81 US 300421**
**28.06.82 US 392139**

(43) Date of publication of application:
**23.03.83 Bulletin 83/12**

(45) Publication of the grant of the patent:
**14.10.87 Bulletin 87/42**

(84) Designated Contracting States:
**AT BE CH DE FR GB IT LI LU NL SE**

(56) References cited:
**GB-A-2 044 255**

(73) Proprietor: **PFIZER INC.**
**235 East 42nd Street**
**New York, N.Y. 10017 (US)**

(72) Inventor: **Kellogg, Michael Stephen**
**26 Lincoln Road Waterford**
**New London Connecticut (US)**

(74) Representative: **Graham, Philip Colin Christison et al**
**Pfizer Limited Ramsgate Road**
**Sandwich, Kent CT13 9NJ (GB)**

**The file contains technical information submitted after the application was filed and not included in this specification**

**EP 0 074 777 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to new chemical compounds which are of value as antibacterial agents. More particularly it relates to certain new bis-esters of methanediol, in which one hydroxy group of the methanediol has been esterified with the carboxy group of a 6-(2-amino-2-[4-acyloxyphenyl]-acetamido)penicillanic acid compound and the other hydroxy group has been esterified with the carboxy group of a beta-lactamase inhibitor. Said latter beta-lactamase inhibitor is one of the type which contains a beta-lactam ring as well as a carboxy group.

United States patent No. 4,244,951, Belgian patent No. 887,173 and published British patent application No. 2,044,255 discloses a variety of bis-esters of methanediol of the formula

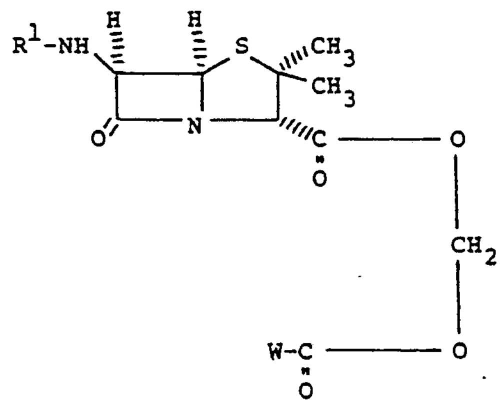

---(I)

and the pharmaceutically-acceptable salts thereof, wherein $R^1$ represents certain acyl groups and the radical W—C(=O)—O— represents a radical of a beta-lactamase inhibitor, W—C(=O)—OH, which contains a beta-lactam ring as well as a carboxy group, said compounds of formula I being useful as antibacterial agents. In particular, $R^1$ can represent a 2-amino-2-(4-hydroxyphenyl)acetyl group. However, it has now been found that compounds of the formula I, wherein the group $R^1$ represents certain 2-amino-2-(4-acyloxyphenyl)acetyl groups, constitute a new genus of bis-esters of methanediol, having outstanding value in the treatment of bacterial infections in mammals. Additionally, compounds of formula I, in which $R^1$ represents certain 2-(protected amino)-2-(4-acyloxypheny)acetyl groups, are useful as intermediates to the antibacterial agents of this invention.

The antibacterial agents of the present invention are efficiently absorbed from the gastrointestinal tract of mammals, and after absorption they are transformed into 6-(2-amino-2-[4-hydroxyphenyl]acetamido)-penicillanic acid (amoxicillin) and a beta-lactamase inhibitor.

6-(2-Amino-2-[4-hydroxyphenyl]acetamido)penicillanic acid and 6-(2-amino-2-[4-acyloxyphenyl]-acetamido)penicillanic acids are known; see further United States patents Nos. 2,985,648, 3,520,876 and 4,053,360. Penicillanic acid 1,1-dioxide is known from United States patent No. 4,234,579.

This invention provides new antibacterial agents of the formula

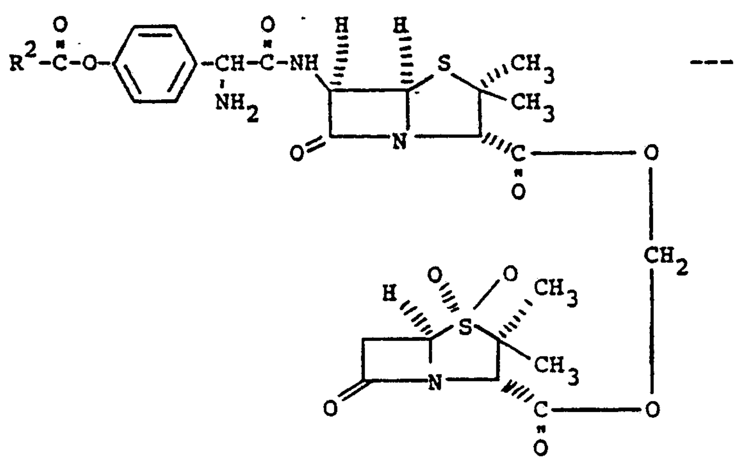

---(II)

and the pharmaceutically acceptable acid addition salt thereof, wherein $R^2$ is alkyl having from one to six carbons.

Such compounds have been found to have unexpected advantages when compared with the unacylated compound disclosed in Example 2 of GB 2044255.

This invention relates to derivatives of penicillanic acid, which is represented by the following structural formula

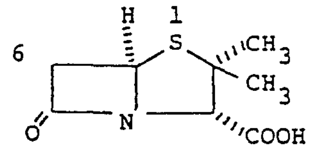

--- (III)

In formula III, broken line attachment of a substituent to the bicyclic nucleus indicates that the substituent is below the plane of the bicyclic nucleus, and such a substituent is said to be in the alpha-configuration. Conversely, solid line attachment of a substituent to the bicyclic nucleus indicates that the substituent is attached above the plane of the nucleus, and this latter configuration is referred to as the beta-configuration. Additionally, wavy line attachment of a substituent to the bicyclic nucleus indicates that the substituent is in the alpha-configuration or the beta-configuration or that a mixture is present.

Thus for example, using this system, the compounds of formulae II are named as derivatives of penicillanoyloxymethyl penicillanate (IV), in which primed and unprimed locants are used to distinguish between the two ring systems, viz:

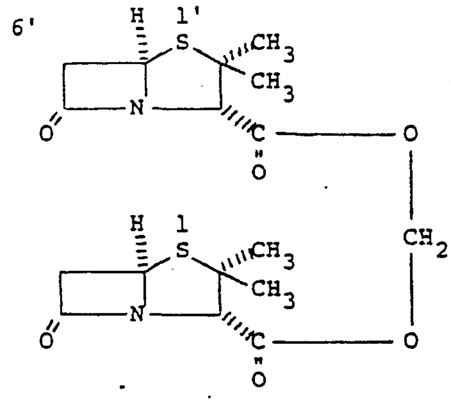

--- (IV)

Additionally, throughout this specification, whenever reference is made to a compound which has a 2-amino-2-(substituted)acetamido or 2-(substituted amino)-2-(substituted)acetamido group at the 6-position of a penicillanic acid derivative, it is to be understood that this refers to a compound in which said 2-amino-2-(substituted)acetamido or 2-(substituted amino)-2-(substituted)acetamido has the the *D*-configuration.

The compounds of formula II can be prepared as follows. The phenolic hydroxy group in the appropriate compound of the formula

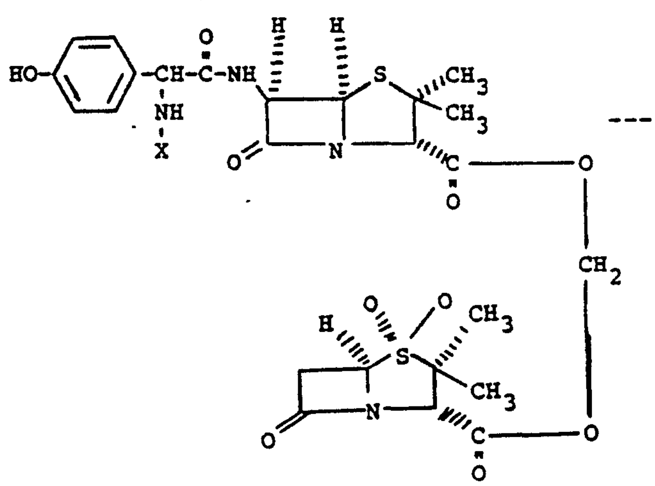

--- (V)

wherein X is an amino protecting group, is acylated with an activated derivative of a carboxylic acid of the formula $R^2$—C(=O)—OH to give the corresponding compound of the formula

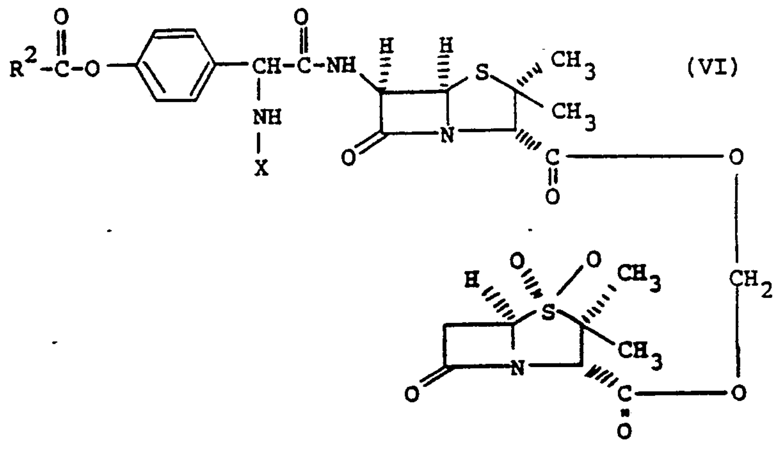

(VI)

This acylation is followed by removal of the protecting group X.

A variety of protecting groups can be used for the group X. Convenient groups for X are 1-methyl-2-alkoxycarbonylvinyl groups having 1 to 3 carbons in said alkoxy moiety, the benzyloxycarbonyl group and the 4-nitrobenzyloxycarbonyl group. 1-Methyl-2-methoxy-carbonylvinyl(—$C[CH_3]=CH—COOCH_3$) is particularly preferred.

The acylation of a compound of formula V can be carried out by reacting said compound of formula V with an acid chloride of the formula $R^2$—CO—Cl or an acid anhydride of the formula $(R^2—CO)_2O$. The acylation reaction is usually conducted in a reaction-inert solvent system. In a typical procedure, from 0.5 to 2.0 molar equivalents, and preferably from 1 molar equivalent, of the acylating agent of formula $R^2$—CO—Cl or $(R^2—CO)_2O$ is contacted with said compound of formula V, in a reaction-inert solvent, in the presence of a tertiary amine, at a temperature in the range from −10 to 30°C. Reaction-inert solvents which can be used in this acylation are: chlorinated hydrocarbons, such as chloroform and dichloromethane; ethers, such as diethyl ether and tetrahydrofuran; low molecular weight esters, such as ethyl acetate and butyl acetate; low molecular weight aliphatic ketones, such as acetone and methyl ethyl ketone; tertiary amides, such as *N,N*-dimethylformamide and *N*-methylpyrrolidone; acetonitrile; and mixtures thereof. The tertiary amine is normally used in an amount equivalent to the compound of formula $R^2$—CO—Cl or $(R^2—CO)_2O$, and typical tertiary amines which can be used are triethylamine, tributylamine, diisopropylethylamine, pyridine and 4-dimethylaminopyridine.

The compounds of formula VI can be isolated by conventional means, such as removal of the solvent by evaporation. They can be purified, if desired, by conventional methods such as recrystallization or chromatography; alternatively, the protecting group X can be removed from the crude acylation product.

The protecting group X is removed from a compound of formula VI by a conventional method for that particular protecting group, but due regard must be given to the lability of the beta-lactam rings and the methylenedioxy linkage.

The 1-methyl-2-alkoxycarbonylvinyl groups can be removed simply by exposing the compound of formula VI to an aqueous or partially aqueous solvent system at an acidic pH, i.e. a pH from 0.5 to 3. This is conveniently achieved by treating the acylation product with water and one molar equivalent of a strong acid, at room temperature, optionally in the presence of a co-solvent. Typical examples of strong acids which can be used are hydrochloric acid, hydrobromic acid, perchloric acid, sulfuric acid, nitric acid and sulfonic acids, such as methanesulfonic acid, benzenesulfonic acid, toluenesulfonic acids and naphthalenesulfonic acids. A variety of co-solvents can be used, the major requirements of such a solvent being that it is at least partially miscible with water and it does not adversely affect either the starting material or the product. Typical co-solvents are low molecular weight ketones, such as acetone and low molecular weight ethers, such as tetrahydrofuran and 1,2-dimethoxyethane. The reaction is usually complete within an hour, and the product is isolated by conventional methods. In many instances, it is sufficient simply to remove the co-solvent by evaporation *in vacuo*, remove the alkyl acetoacetate by extraction with a water-immiscible solvent such as diethyl ether, and then lyophilize the remaining aqueous solution. This affords the requisite compound of formula II as a salt corresponding to the acid which has been added initially.

The benzyloxycarbonyl and 4-nitrobenzyloxycarbonyl groups can be removed from a compound of formula VI by catalytic hydrogenolysis. In this case, a compound of formula VI, wherein X is benzyloxycarbonyl or 4-nitrobenzyloxycarbonyl, is stirred or shaken under an atmosphere of hydrogen, or hydrogen mixed with an inert diluent such as nitrogen or argon, in the presence of a catalytic amount of a hydrogenolysis catalyst. Convenient solvents of this hydrogenolysis are lower-alkanols, such as methanol and isopropanol; ethers, such as tetrahydrofuran and dioxan; low molecular weight esters, such as ethyl acetate and butyl acetate; chlorinated hydrocarbons, such as dichloromethane and chloroform; water; and

mixtures of these solvents. However, it is usual to choose conditions under which the starting material is soluble. The hydrogenolysis is usually carried out at a temperature in the range from 0 to 60°C and at a pressure in the range from 20 to 100 psig, preferably about 50 psig. The catalysts used in this hydrogenolysis reaction are the type of agents known in the art for this kind of transformation, and typical examples are the noble metals, such as nickel, palladium, platinum and rhodium. It is often convenient to suspend the catalyst on an inert support; a particularly convenient catalyst is palladium suspended on an inert support such as carbon, e.g. 10% palladium on carbon. When 10% palladium on carbon is used, it is usually used in a weight amount that is 0.5 to 5.0, and preferably about 1.0, times the weight of the compound of formula VI.

The compounds of the formula II will form acid addition salts, and these acid addition salts are considered to be within the scope and purview of this invention. Said acid addition salts are prepared by standard methods for penicillin compounds, for example by combining a solution of the compound of formula II in a suitable solvent (e.g. water, ethyl acetate, acetone, methanol, ethanol or butanol) with a solution containing a stoichiometric equivalent of the appropriate acid. If the salt precipitates, it is recovered by filtration. Alternatively, it can be recovered by evaporation of the solvent, or, in the case of aqueous solutions, by lyophilization. Of particular value are the sulfate, hydrochloride, hydrobromide, nitrate, phosphate, citrate, tartrate, pamoate, perchlorate, sulfosalicylate benzenesulfonate, 4-toluenesulfonate and 2-naphthalenesulfonate salts.

When contemplaing therapeutic use for a salt of an antibacterial compound of this invention, it is necessary to use a pharmaceutically-acceptable salt; however, salts other than these can be used for a variety of purposes. Such purposes include isolating and purifying particular compounds, and interconverting pharmaceutically-acceptable salts and their non-salt counterparts.

The compounds of the formula II, and the salts thereof, can be purified by conventional methods for penicillin compounds, e.g. recrystallization or chromatography, but due regard must be given to the lability of the beta-lactam ring systems and the methylenedioxy linkage.

Those compounds of formula V which are known are prepared by the known method, and those compounds of formula V which are analogs of known compounds are prepared by methods which are analogous to the known methods. See United States patent No. 4,244,951, published British patent application No. 2,044,255, published Dutch patent application No. 81/00209 and Belgian patent No. 887,173.

The compounds of formula II possess *in vivo* antibacterial activity in mammals, and this activity can be demonstrated by standard techniques for penicillin compounds. For example, the compound of formula II is administered to mice in which acute infections have been established by intraperitoneal inoculation with a standardized culture of a pathogenic bacterium. Infection severity is standardized such that the mice receive one to ten times the $LD_{100}$ ($LD_{100}$: the minimum inoculation required to consistently kill 100 percent of control mice). At the end of the test, the activity of the compound is assessed by counting the number of survivors which have been challenged by the bacterium and also have received the compound of formula II. The compounds of formula II can be administered by both the oral (p.o.) and subcutaneous (s.c.) route.

The *in vivo* activity of the antibacterial compounds of this invention makes them suitable for the control of bacterial infections in mammals, including man, by both the oral and parenteral modes of administration. The compounds are useful in the control of infections caused by susceptible bacteria in human subjects.

A compound of formula II breaks down to 6-(2-amino-2-[4-hydroxyphenyl]acetamido)penicillanic acid (amoxicillin) and penicillanic acid 1,1-dioxide (sulbactam), after administration to a mammalian subject by both the oral and parenteral route. Sulbactam then functions as a beta-lactamase inhibitor, and it increases the antibacterial effectiveness of the amoxicillin. Thus the compounds of the formula II will find use in the control of. bacteria which are susceptible to a 1:1 mixture of amoxicillin and sulbactam e.g. susceptible strains of *Escherichia coli* and *Straphylococcus aureus*.

In determining whether a particular strain of *Escherichia coli* or *Straphylococcus aureus* is sensitive to a particular compound of formula II, the *in vivo* test described earlier can be used. Alternatively, the minimum inhibitory concentration (MIC) of a 1:1 mixture of amoxicillin and sulbactam can be measured. The MIC's can be measured by the procedure recommended by the Internatinal Collaborative Study on Antiobiotic Sensitivity Testing (Ericcson and Sherris, *Acta. Pathologica et Microbiologia* Scandinav, Supp. 217, Section B: 64—68 [1971]), which employs brain heart infusion (BHI) agar and the inocula replicating device. Overnight growth tubes are diluted 100 fold for use as the standard inoculum (20,000—10,000 cells in approximately 0.002 ml. are placed on the agar surface; 20 ml of BHI agar/dish). Twelve 2 fold dilutions of the test compound are employed, with initial concentration of the test drug being 200 mcg./ml. Single colonies are disregarded when reading plates after 18 hrs. at 37°C. The suspectibility (MIC) of the test organism is accepted as the lowest concentration of compound capable of producing complete inhibition of growth as judged by the naked eye.

When using an antibacterial compound of this invention, or a salt thereof, in a mammal, particularly man, the compound can be administered alone, or it can be mixed with other antibiotic substances and/or pharmaceutically-acceptable carriers or diluents. Said carrier or diluent is chosen on the basis of the intended mode of administration. For example, when considering the oral mode of administration, an antibacterial compound of this invention can be used in the form of tablets, capsules, lozenges, troches, powders, syrups, elixirs, aqueous solutions and suspensions, and the like, in accordance with standard

pharmaceutical practice. The proportional ratio of active ingredient to carrier will naturally depend on the chemical nature, solubility and stability of the active ingredient, as well as the dosage contemplated. In the case of tablets for oral use, carriers which are commonly used include lactose, sodium citrate and salts of phosphoric acid. Various disintegrants such as starch, and lubricating agents, such as magnesium stearate, sodium lauryl sulfate and talc, are commonly used in tablets. For oral administration in capsule form, useful diluents are lactose and high molecular weight polyethylene glycols, e.g. polyethylene glycols having molecular weights of from 2000 to 4000. When aqueous suspensions are required for oral use, the active ingredient is combined with emulsifying and suspending agents. If desired, certain sweetening and/or flavoring agents can be added. For parenternal administration, which includes intramuscular, intraperitoneal, subcutaneous, and intravenous use, sterile solutions of the active ingredient are usually prepared, and the pH of the solutions are suitably adjusted and buffered. For intravenous use, the total concentration of solutes should be controlled to render the preparation isotonic.

As indicated earlier, the antibacterial compounds of this invention are of use in human subjects and the daily dosages to be used will not differ significantly from other, clinically-used, penicillin antibiotics. The prescribing physician will ultimately determine the appropriate dose for a given human subject, and this can be expected to vary according to the age, weight, and response of the individual patient as well as the nature and the severity of the patient's symptoms. The compounds of this invention will normally be used orally at dosages in the range from 20 to about 100 mg. per kilogram of body weight per day, and parenterally at dosages from about 10 to about 100 mg per kilogram of body weight per day, usually in divided doses. In some instances it may be necessary to use doses outside these ranges.

The following examples and preparations are provided solely for further illustration. Nuclear magnetic resonance spectra (NMR) were measured for solutions in deuterated chloroform ($CDCl_3$) or deuterated dimethyl sulfoxide (DMSO-$d_6$), and peak positions are reported in parts per million downfield from tetramethylsilane. The following abbreviations for peak shapes are used: bs, broad singlet; s, singlet; d, doublet; t, triplet, q, quartet, m, multiplet.

Example 1

6'-(2-Amino-2-[4-isobutyryloxyphenyl]acetamido)penicillanoyloxymethyl Penicillanate 1,1-Dioxide Hydrochloride

To a stirred solution of 1.5 g of 6'-(2-[1-methyl-2-methoxycarbonylvinylamino]-2-[4-isobutyryloxyphenyl]acetamido)penicillanoyloxymethyl penicillanate 1,1-dioxide in 30 ml of acetone was added 20 ml of 0.1N hydrochloric acid. Stirring was continued for 5 minutes and then the acetone was removed by evaporation *in vacuo*. The remaining aqueous solution was extracted twice with 30 ml portions of diethyl ether. The extracts were discarded, and the aqueous layer was filtered through Celite (a diatomaceous silica product). The filtrate was lyophilized to give 1.09 g of the title compound as a solid.

The NMR spectrum of the product (in DMSO-$d_6$) showed absorptions at 1.20—1.60 (m, 18H), 2.64—3.00 (m, 1H), 3.05—3.92 (m, 2H), 4.40 (s, 1H), 4.50 (s, 1H), 5.05—5.30 (m, 2H), 5.32—5.60 (m, 2H), 5.85 (s, 2H), 7.10 (d, 2H) and 7.50 (d, 2H) ppm.

Example 2

Hydrolysis of the products of Examples 5 and 7—9 with 0.1N hydrochloric acid, substantially according to the procedure of Example 1, afforded the following compounds

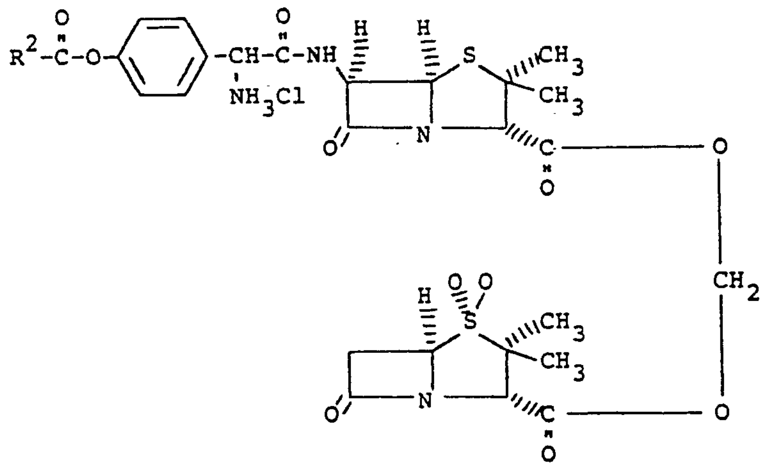

| $R^2$ | Yield (%) | NMR; DMSO-$d_6$; ppm |
|---|---|---|
| methyl | 75 | 1.20—1.64 (m, 12H), 2.28 (s, 3H) 3.00—3.90 (m, 2H), 4.43 (s, 1H), 4.50 (s, 1H), 5.03—5.30 (m, 2H), 5.30—5.60 (m, 2H), 5.90 (s, 2H), 7.18 (d, 2H) and 7.55 (d, 2H) |
| ethyl | 71 | 1.19 (t, 3H) 1.42 (s, 6H), 1.52 (s, 6H), 2.62 (q, 2H), 3.08—3.92 (m, 2H), 4.42 (s, 1H), 5.52 (s, 1H), 5.08—5.18 (m, 2H), 5.35—5.60 (m, 2H), 5.89 (s, 2H), 7.15 (d, 2H), 7.52 (d, 2H) |
| *t*-butyl | 83 | 1.15—1.60 (m, 21H), 3.05—3.90 (m, 2H), 4.48 (s, 1H), 4.55 (s, 1H), 5.05—5.30 (m, 2H), 5.40—5.70 (m, 2H), 5.92 (s, 2H), 7.18 (d, 2H), 7.6o (d, 2H), 8.60—9.30 (bs, 3H), 9.50 (s, 1H) |
| *t*-butylmethyl | 82 | 1.08 (s, 9H), 1.38 (s, 6H), 1.44 (s, 6H), 2.44 (s, 2H), 3.00—3.90 (m, 2H), 4.40 (s, 1H), 4.52 (s, 1H), 5.05—5.30 (m, 2H), 5.32—5.60 (m, 2H), 5.88 (bs, 2H), 7.10 (d, 2H), 7.58 (d, 2H) |

Example 3

6'-(2-Amino-2-[4-acetoxyphenyl]acetamido)penicillanoyloxymethyl Penicillanate 1,1-Dioxide

A mixture of 1.9 g of 6'-(2-benzyloxycarbonylamino-2-[4-acetoxyphenyl]acetamido)penicillanoyloxymethyl penicillanate 1,1-dioxide, 50 ml of dichloromethane, 50 ml of isopropanol and 2.0 g of 10% palladium-on-carbon was shaken under an atmosphere of hydrogen at *ca* 45 psig for 20 minutes. At this point an additional 2.0 g of palladium-on-carbon was added and the mixture was shaken under hydrogen for 20 minutes at *ca* 45 psig. The procedure of adding an additional 2.0 g of palladium-on-carbon and

shaking under hydrogen at *ca* 45 psig and then repeated four more times. The reaction mixture was then filtered through Celite (a diatomaceous silica product) and the residue (filter cake) was washed well with 1:1 dichloromethaneisopropanol. The combined filtrate and washings were evaporated *in vacuo* to give a white solid, which was triturated under diethyl ether. This afforded 0.3 g of a first crop of the title compound.

The above filter cake was washed successively with 100 ml portions of acetone, dichloromethane and isopropanol. The combined washings were concentrated *in vacuo* to give a grey solid. The solid was triturated under diethyl ether. This afforded 0.2 g of a second crop of the title compound.

The NMR spectrum of the first crop of the title compound (in DMSO-$d_6$) showed absorptions at 1.35 (s, 6H), 1.48 (s, 6H), 2.28 (s, 3H), 3.00—3.90 (m, 2H), 4.42 (s, 1H), 4.50 (s, 1H), 5.00 (bs, 1H), 5.05—5.20 (m, 1H), 5.34—5.58 (m, 2H), 5.90 (s, 2H), 7.10 (d, 2H) and 7.54 (d, 2H) ppm.

Example 4

6′-(2-Amino-2-[4-acetoxyphenyl]-penicillanoyloxymethyl Penicillanate 1,1-Dioxide Hydrochloride

The two crops of 6′-(2-amino-2-[4-acetoxyphenyl]acetamido)penicillanoyloxymethyl penicillanate 1,1-dioxide from Example 3 were combined and added to a cold (0°C), stirred mixture which had been prepared from 20 ml of water and 6.7 ml of 0.1*N* hydrochloric acid. Stirring was continued for 15 minutes and then the mixtures were filtered. The filtrate was lyophilized to give 0.34 g of the title salt.

Example 5

6′-(2-[1-Methyl-2-methoxycarbonylvinylamino]-2-[4-acetoxyphenyl]acetamido)penicillanoyloxymethyl Penicillanate 1,1-Dioxide

To a stirred solution of 3.5 g of 6′-(2-[1-methyl-2-methoxycarbonylvinylamino]-2-[4-hydroxyphenyl]acetamido)penicillanoyloxymethyl penicillanate 1,1-dioxide and 0.61 g of 4-dimethylaminopyridine, in 30 ml of dichloromethane, wsa added 0.47 ml of acetic anhydride. Stirring was continued for 30 minutes, and then the reaction mixture was concentrated to half volume. The latter solution was then diluted with an equal volume of ethyl acetate and chromatographed on 100 g of silica gel using 1:1 ethyl acetate-dichloromethane as eluant. The appropriate fractions were combined and evaporated *in vacuo* to give 2.7 g of the title compound as a light orange foam.

Example 6

6′-(2-[1-Methyl-2-methoxycarbonylvinylamino]-2-[4-isobutyryloxyphenyl]acetamido)penicillanoyloxymethyl Penicillanate 1,1-Dioxide

To a stirred solution of 2.12 g of 6′-(2-[1-methyl-2-methoxycarbonylvinyl]-2-[4-hydroxyphenyl]-acetamido)penicillanoyloxymethyl penicillanate 1,1-dioxide and 0.366 g of 4-dimethylaminopyridine in 30 ml of dichloromethane was added 0.314 ml of isobutyryl chloride. Stirring was continued for 30 minutes and then an additional 75 ml of dichloromethane was added. The mixture was washed successively with water and saturated sodium chloride solution, and then it was dried using sodium sulfate. The solvent was removed by evaporation *in vacuo*, and the residue was chromatographed on 150 g of silica gel, using 60:40 dichloromethane-ethyl acetate as eluant. This afforded 1.5 g of the title compound as a colorless foam.

Example 7

6'-(2-[1-Methyl-2-methoxycarbonylvinylamino]-2-[4-hydroxyphenyl]acetamido)penicillanoyloxymethyl-penicillanate 1,1-dioxide was acylated with propionyl chloride, pivaloyl chloride and *t*-butylacetyl chloride, respectively, substantially according to the procedure of Example 6. This afforded the following compounds

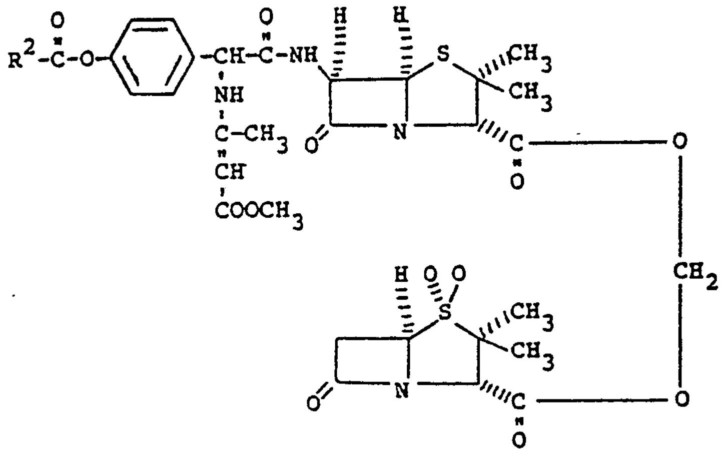

| $R^2$ | Yield (%) | NMR (in ppm) |
|---|---|---|
| ethyl | 52* | — |
| *t*-butyl | 91 | 1.18—1.56 (m, 21H), 1.78 (s, 3H), 3.00—3.90 (m, 2H), 3.54 (s, 3H), 4.43 (s, 1H), 4.48 (s, 1H), 4.52 (s, 1H), 5.08—5.23 (m, 1H), 5.30—5.65 (m, 3H), 5.90 (s, 2H) 5.07 (d, 2H) and 7.40 (d, 2H) ppm ( DMSO-$d_6$) |
| *t*-butylmethyl | 56 | 1.17 (s, 9H), 1.44 (s, 3H), 1.48 (s, 3H), 1.55 (s, 3H), 1.62 (s, 3H), 1.92 (s, 3H), 2.46 (s, 2H), 3.47 (d, 2H), 3.68 (s, 3H), 4.40 (s, 1H), 4.42 (s, 1H), 4.50—4.68 (m, 2H), 5.10 (d, 2H), 5.35—5.60 (m, 2H), 5.85 (s, 2H), 6.77 (d, 1H), 7.06 (d, 2H) and 7.40 (d, 2H) ppm ($CDCl_3$) |

* This product was not chromatographed.

Example 8

6'-(2-[Benzyloxycarbonylamino]-2-[4-acetoxyphenyl]acetamido)penicillanoyloxymethyl Penicillanate 1,1-Dioxide

To a stirred solution of 2.23 g of 6'-(2-benzyloxycarbonylamino-2-[4-hydroxyphenyl]acetamido)-penicillanoyloxymethyl penicillanate 1,1-dioxide in 50 ml of dichloromethane was added 0.28 ml of acetic anhydride followed by 0.366 g of 4-dimethylaminopyridine. Stirring was continued for 10 minutes and then the solvent was removed by evaporation *in vacuo*. The residue was dissolved in ethyl acetate, and the solution obtained was washed with water. It was then dried using sodium sulfate and concentrated *in vacuo* to give 2.0 g of the title compound as a foam.

The NMR spectrum (in $CDCl_3$) showed absorptions at 1.35—1.60 (m, 12H), 2.25 (s, 3H), 3.40 (d, 2H), 4.38 (s, 2H), 4.56 (t, 1H), 5.04 (s, 2H), 5.20—5.60 (m, 3H), 5.80 (s, 2H), 6.12 (d, 1H), 6.48 (d, 2H), 7.24 (s, 5H) and 7.30 (d, 2H) ppm.

Example 9

6'-(2-Amino-2-[4-butyryloxyphenyl]acetamido)penicillanoyloxymethyl Penicillanate 1,1-Dioxide 4-Toluenesulfonate

To a stirred solution of 7.1 g of 6'-(2-[1-methyl-2-methoxycarbonylvinylamino]-2-[4-hydroxyphenyl]acetamido)penicillanoyloxymethyl penicillanate 1,1-dioxide in 75 ml of ethyl acetate was added 1.22 g of 4-dimethylaminopyridine followed by 1.63 ml of butyric anhydride. Stirring was continued for 20 minutes and then the reaction medium was diluted with ethyl acetate to 125 ml and washed with water and with saturated sodium chloride solution. The solution obtained was dried using sodium sulfate, and then a solution of 1.9 g of 4-toluenesulfonic acid monohydrate in 35 ml of ethyl acetate and 1 ml of water was added, with stirring, during 2 minutes. After 30 minutes, the precipitate was recovered by filtration, and then it was washed with ethyl acetate and air dried. The product was then triturated under diethyl ether and further dried. This afforded 6.2 g of the title compound.

The NMR spectrum of the product (DMSO-$d_6$) showed absorptions at 1.03 (t, 2H), 1.3—1.9 (m, 14H), 2.33 (s, 3H), 2.4—2.8 (m, 2H), 3.1—3.9 (m, 2H), 4.46 (s, 1H), 4.56 (s, 1H), 5.06—5.3 (m, 2H), 5.4—5.66 (m, 2H), 5.93 (bs, 2H), 7.0—7.36 (m, 4H) and 7.4—7.66 (m, 4H) ppm.

The IR spectrum of the product (nujol mull) showed an absorption at 1790 $cm^{-1}$.

Preparation 1

6′-(2-[1-Methyl-2-methoxycarbonylvinylamino]-2-[4-hydroxyphenyl]acetamido)penicillanoyloxymethyl Penicillanate 1,1-Dioxide

To 300 ml of dichloromethane was added 41.9 g of 6-(2-amino-2-[4-hydroxyphenyl]acetamido)-penicillanic acid trihydrate and 50 ml of water, and then the pH was adjusted to 8.5 using 40% aqueous tetra-n-butyl-ammonium hydroxide. Three layers were obtained. The upper layer was removed, saturated with sodium sulfate and then it was extracted with dichloromethane. The extracts were combined with the middle layer and the lower layer, and the resulting mixture was evaporated *in vacuo* to give an oil which crystallized on trituration with acetone. This afforded 44.6 g of tetra-n-butylammonium 6-(2-amino-2-[4-hydroxyphenyl]acetamido)penicillanate.

The above salt was added to 150 ml of methyl acetoacetate and the suspension was heated at *ca.* 65°C until a clear solution was obtained (8 minutes). The mixture was allowed to cool, and then the solid was recovered by filtration. The solid was washed with methyl acetoacetate, followed by diethyl ether, to give 49.25 g of tetra-n-butylammonium 6-(2-[1-methyl-2-methoxycarbonylvinylamino]-2-[4-hydroxyphenyl]-acetamido)penicillanate.

To 47.5 g of the latter product in 250 ml of dimethylformamide at 0°C was added, with stirring, 18.26 g of iodomethyl penicillanate 1,1-dioxide in 50 ml of the same solvent, over a 20 minute period. Ten minutes after completion of the addition, the reaction mixture was poured into 3 l of ethyl acetate and the resulting precipitate was filtered off. The precipitate was washed with ethyl acetate (100 ml), and then the combined ethyl acetate solution was washed successively with a brine solution (4 × 500 ml), water (4 × 500 ml) and a brine solution (2 × 500 ml) and dried over sodium sulfate. The residue remaining after the solvent was removed was chromatographed over 750 g of silica gel using ethyl acetate as the eluant. The fractions (250 ml each) 2—5 were combined and concentrated to give 31.2 g of the title compound.

The NMR spectrum (DMSO-$d_6$, $^1H$ 100.1M Hz) showed absorptions at 1.37 (s, 3H), 1.38 (s, 3H), 1.48 (s, 3H), 1.57 (s, 3H), 1.76 (s, 3H), 3.14—3.82 (m, 2H), 3.51 (s, 3H), 4.42 (s, 1H), 4.44 (s, 1H), 4.54 (s, 1H), 5.1—5.22 (m, 1H), 5.3—5.64 (m, 2H), 5.9 (s, 2H), 6.7 (d, 2H), 7.14 (d, 2H), 9.02 (d, 1H), 9.24 (d, 1H) and 9.34—9.54 (bs, 1H) ppm.

Preparation 2

6′-(2-Benzyloxycarbonylamino-2-[4-hydroxyphenyl]acetamido)penicillanoyloxymethyl Penicillanate 1,1-Dioxide

To 9.5 g of tetra-n-butylammonium 6-(2-benzyloxycarbonylamino-2-[4-hydroxyphenyl]acetamido)-penicillanate in 50 ml of dry acetone was added 4.78 g of iodomethyl penicillanate 1,1-dioxide, and the reaction mixture allowed to stir at room temperature for 30 min. The reaction mixture was concentrated *in vacuo* and the residue was chromatographed on 200 g of silica gel using ethyl acetate/dichloromethane (1:1 v/v), 25 ml, cuts being made. Fractions 29—49 were combined and concentrated to give 6.5 g of the desired product as a yellow foam.

The NMR spectrum (DMSO-$d_6$) showed absorptions at 1.42 (s, 3H), 1.52 (s, 3H), 1.6 (s, 3H), 3.1—3.9 (m, 2H), 4.45 (s, 1H), 4.58 (s, 1H), 5.08 (s, 2H), 4.98—5.7 (m, 4H), 5.95 (s, 2H), 6.68 (d, 2H), 7.2 (d, 2H) and 7.35 (s, 5H) ppm.

Preparation 3

Tetra-n-butylammonium 6-(2-Benzyloxycarbonylamino-2-[4-hydroxyphenyl]acetamido)penicillanate

To a rapidly stirred mixture of 1.0 g of 6-(2-benzyloxycarbonylamino-2-[4-hydroxyphenyl]acetamido)-penicillanic acid, 30 ml of dichloromethane and 20 ml of water was added 40% aqueous tetra-n-butylammonium hydroxide until a pH of 8.0 was obtained. Stirring was continued for 30 minutes at pH 8.0 and then the layers were separated. The aqueous layer was extracted with dichloromethane, and then the combined dichloromethane solutions were dried ($Na_2SO_4$) and evaporated *in vacuo*. This afforded 1.1 g of the title compound.

The NMR spectrum (in DMSO-$d_6$) showed absorptions at 0.70—1.80 (m, 34H), 2.90—3.50 (m, 8H), 3.93 (s, 1H), 5.10 (s, 2H), 5.23—5.50 (m, 3H), 6.76 (d, 2H), 7.20 (d, 2H), 7.40 (s, 5H), 7.76 (d, 1H) and 8.6 (d, 1H) ppm.

Preparation 4

Chloromethyl Penicillanate 1,1-Dioxide

A mixture of 4.66 g of penicillanic acid 1,1-dioxide, 50 ml of dichloromethane and 35 ml of water was treated with sufficient tetra-n-butylammonium hyroxide (40% in water) to give a pH of 6.0. The dichloromethane layer was separated and the aqueous phase extracted with fresh dichloromethane (2 × 50 ml). The organic layers were combined, dried over sodium sulfate and concentrated to give 10.1 g of the tetra-n-butylammonium salt of penicillanic acid 1,1-dioxide.

The above tetra-n-butylammonium penicillanate 1,1-dioxide was added to 50 ml of chloroiodomethane and the reaction mixture allowed to stir at ambient temperature overnight. The reaction mixture was concentrated to half volume *in vacuo*, and chromatographed on 200 g of silica gel using ethyl acetate/hexane as the eluant, 12 ml cuts being taken every 30 seconds. Fractions 41—73 were combined and concentrated to dryness to give 3.2 g of the title compound.

The NMR spectrum ($CDCl_3$) showed absorptions at 1.5 (s, 3H), 1.66 (s, 3H), 3.42 (d, 2H), 4.38 (s, 1H), 4.6 (t, 1H) and 5.7 (dd, 2H) ppm.

Preparation 5

Iodomethyl Penicillanate 1,1-Dioxide

To a solution of 7.9 g of chloromethyl penicillanate 1,1-dioxide in 100 ml of dry acetone maintained under a nitrogen atmosphere was added 21.0 g of sodium iodide, and the reaction mixture was allowed to stir overnight at room temperature. The reaction mixture was concentrated *in vacuo*, and the residue was dissolved in 150 ml ethyl acetate and 150 ml water. The organic layer was separated and the aqueous layer was extracted with fresh ethyl acetate. The organic extracts were combined, washed with water (1 × 500 ml) and brine (1 × 50 ml) and dried over sodium sulfate. Removal of the solvent gave 10.5 g of the title product, m.p. 100—102°C.

The NMR spectrum ($CDCl_3$) showed absorptions at 1.55 (s, 3H), 1.68 (s, 3H), 3.5 (d, 2H), 4.4 (s, 1H), 4.65 (t, 1H) and 6.0 (dd, 2H) ppm.

Preparation 6

Mono Benzyl Ester Mono Acid Chloride of Dimethylmalonic Acid

A mixture of 1.0 g of the mono benzyl ester of dimethylmalonic acid, 1.0 ml of thionyl chloride and 1.5 ml of dichloromethane is stirred at room temperature for 1 hour and then it was heated under reflux for 4 hours. The volatile components are then removed by evaporation *in vacuo* to afford the title compound, which is used directly to acylate a compound of formula X.

Preparation 7

Mono Benzyl Ester of Dimethylmalonic Acid

A solution of 3.12 g (4.8 mmole) of 85% potassium hydroxide in 75 ml benzyl alochol was added to 15.0 g dibenzyl dimethylmalonate in 75 ml benzyl alcohol. The resulting solution was stirred for 60 hours, 1.5 liters of ethyl ether added and the resulting mixture extracted twice with 100 ml portions of water. The combined aqueous layers were washed with 100 ml ether. To the aqueous layer was added 100 ml ethyl ether and the mixture was acidified to pH 2.5 with 6N hydrochloric acid. The ether layer was separated and the aqueous phase extracted again with ether. The ether extracts were dried ($Na_2SO_4$) and solvent evaporated to afford the product as a colorless oil, 8.6 g (81%).

Preparation 8

Dibenzyl Dimethylmalonate

To 75 ml water containing 4.0 g sodium hydroxide was added at 0°C, 17.0 g (0.05 mole) tetrabutylammonium hydrogen sulfate, the mixture was stirred 15 minutes, allowed to warm and 100 ml chloroform containing 14.2 g (0.05 mole) dibenzyl malonate and 6.6 ml (0.10 mole) methyl iodide was added. The mixture (initial pH >12) was stirred for 30 minutes at which time the mixture was pH *ca.* 8. Stirring was continued for ten minutes, the organic phase was separated. To the organic layer was added another charge of 4.0 g sodium hydroxide, 17.0 g tetrabutylammonium hydrogen sulfate in 75 ml water and 6.6 g methyl iodide. The resulting mixture was stirred at room temperature for 30 minutes, the chloroform layer was separated, dried ($Na_2SO_4$) and concentrated *in vacuo*. The resulting residual oil was triturated with 500 ml ethyl ether, the resulting solids were filtered, washed well with ether and the filtrate and washings evaporated to afford 15.0 g (96%) of product.

**Claims for the Contracting State: BE CH DE FR GB IT LI LU NL SE**

1. A pharmaceutically-active compound of the formula

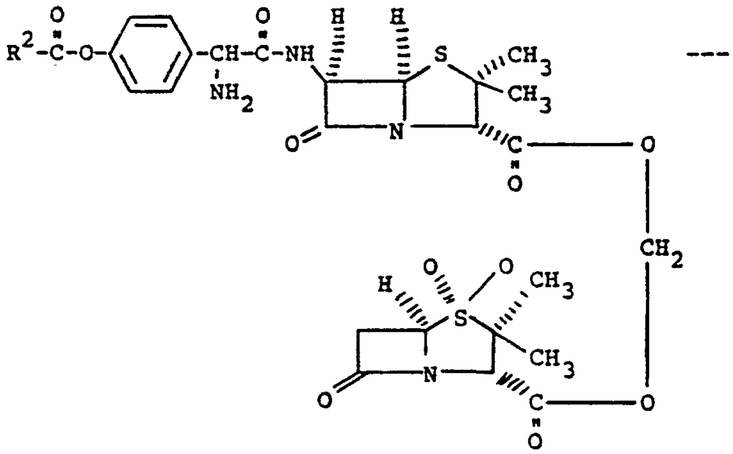

---(II)

and the pharmaceutically-acceptable acid addition salts thereof, wherein $R^2$ is alkyl having from one to six carbons.

2. A compound according to claim 1, characterized in that $R^2$ is isopropyl.

3. A pharmaceutical composition, suitable for treating a bacterial infection in a mammalian subject, characterized in that it comprises as antibacterially-effective amount of a compound according to claim 1 and a pharmaceutically-acceptable carrier.

4. A process for preparing a compound as claimed in Claim 1, characterized in that it comprises removing the amino protecting group X from a compound of the formula

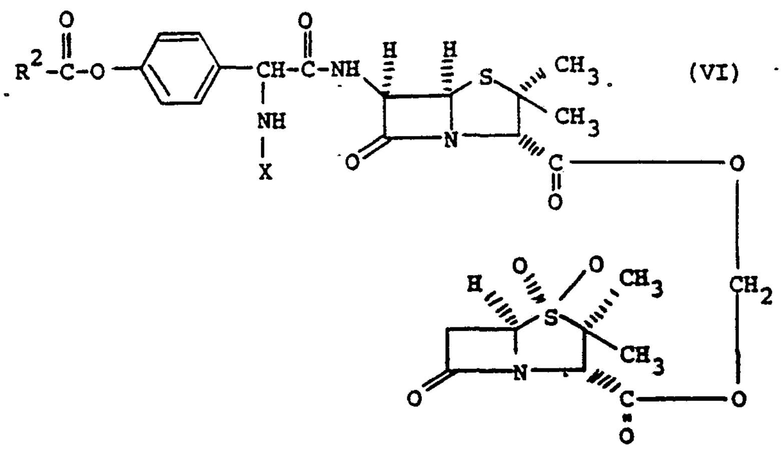

(VI)

5. A process according to Claim 4, wherein X is 1-methyl-2-alkoxycarbonylvinyl ahving 1 to 3 carbons in said alkoxy moiety, benzyloxycarbonyl or 4-nitro-benzyloxycarbonyl.

**Claims for the Contracting State: AT**

1. A process for preparing a pharmaceutically active compound of the formula

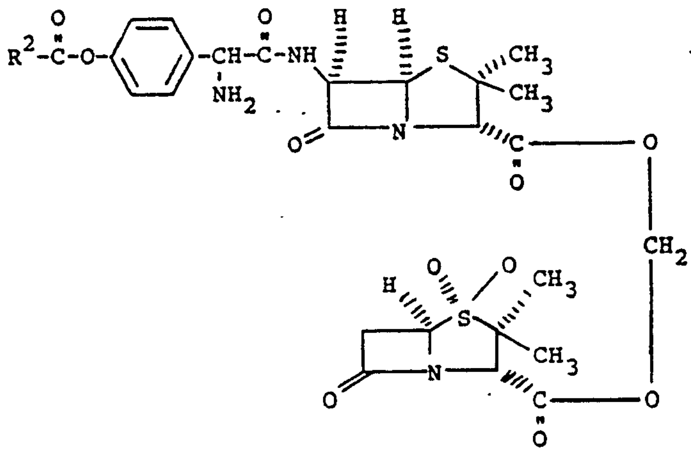

---(II)

or a pharmaceutically-acceptable acid addition salt thereof, wherein $R^2$ is alkyl having from one to six carbons; characterized in that it comprises removing the amino protecting group X from a compound of the formula

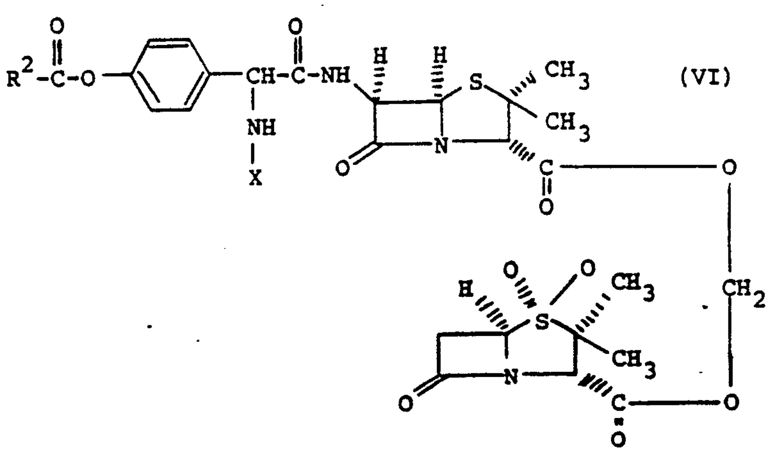

(VI)

2. A process according to claim 1, characterized in that $R^2$ is isopropyl.
3. A process according to claim 1 or claim 2, characterized in that X is 1-methyl-2-alkoxycarbonylvinyl (having 1 to 3 carbons in said alkoxy), benzyloxycarbonyl or 4-nitro-benzyloxycarbonyl.
4. A process according to claim 3, characterized in that X is said 1-methyl-2-alkoxycarbonylvinyl and it is removed by exposing the compound of formula VII to an aqueous or partially aqueous solvent system at an acidic pH.

**Patentansprüche für die Vertragsstaaten: BE CH DE FR GB IT LI LU NL SE**

1. Eine pharmazeutisch aktive Verbindung der Formel

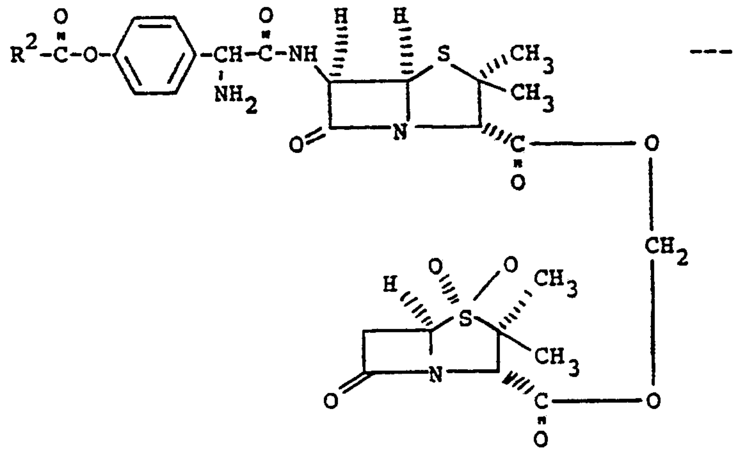

---(II)

und die pharmazeutisch verwendbaren Säureadditionssalze davon, worin $R^2$ Alkyl mit einem bis sechs Kohlenstoffatomen ist.

2. Eine Verbindung gemäß Anspruch 1, dadurch gekennzeichnet, daß $R^2$ Isopropyl ist.

3. Eine pharmazeutische Zusammensetzung, geeignet zur Behandlung einer bakteriellen Infektion in Säugern, dadurch gekennzeichnet, daß sie eine antibakteriell wirksame Menge einer Vrbindung gemäß Anspruch 1 und ein pharmazeutisch verwendbares Trägermaterial enthält.

4. Ein Verfahren zur Herstellung einer Verbindung, wie in Anspruch 1 beansprucht, dadurch gekennzeichnet, daß es die Entfernung der Aminoschutzgruppe X von einer Verbindung der Formel

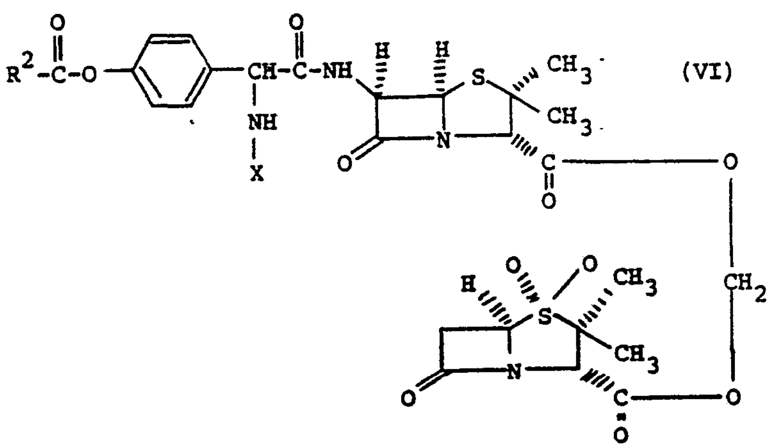

(VI)

umfaßt.

8. Ein Verfahren gemäß Anspruch 4, worin X 1-Methyl-2-alkoxycarbonylvinyl mit 1 bis 3 Kohlenstoffatomen in diesem Alkoxyrest, Benzoyloxycarbonyl oder 4-Nitro-benzyloxycarbonyl ist.

**Patentansprüche für den Vertragsstaat: AT**

1. Ein Verfahren zur Herstellung einer pharmazeutisch aktiven Verbindung der Formel

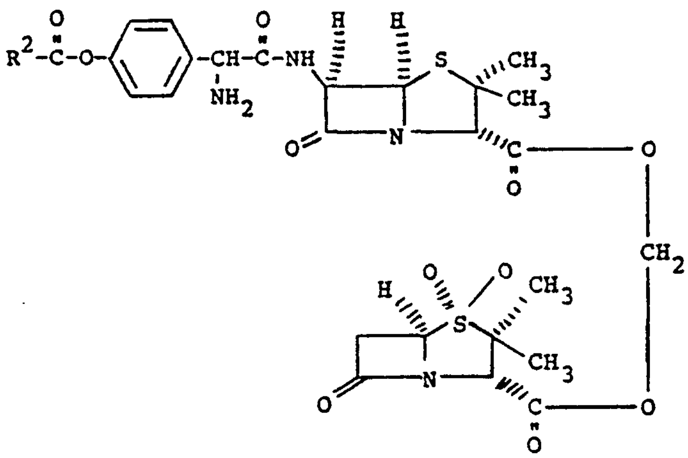

---(II)

oder eines pharmazeutisch verwendbaren Säureadditionssalzes davon, worin $R^2$ Alkyl mit einem bis sechs Kohlenstoffatomen ist; dadurch gekennzeichnet, daß es die Entfernung der Aminoschutzgruppe X von einer Verbindung der Formel

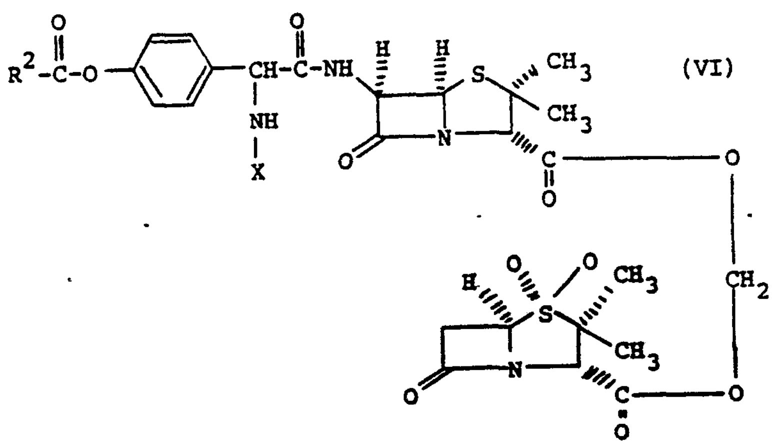

(VI)

umfaßt.

2. Ein Verfahren gemäß Anspruch 1, dadurch gekennzeichnet, daß $R^2$ Isopropyl ist.

3. Ein Verfahren gemäß Anspruch 1 oder Anspruch 2, dadurch gekennzeichnet, daß X 1-Methyl-2-alkoxycarbonylvinyl (mit 1 bis 3 Kohlenstoffatomen in diesem Alkoxy), Benzoyloxycarbonyl oder 4-Nitrobenzyloxycarbonyl ist.

4. Ein Verfahren gemäß Anspruch 3, dadurch gekennzeichnet, daß X dieses 1-Methyl-2-alkoxycarbonylvinyl ist und daß es entfernt wird, indem die Verbindung von Formel VII ein wäßrigen oder teilweise wäßrigem Lösungsmittelsystems bei einem sauren pH ausgesetzt wird.

**Revendications pour les Etats Contractants: BE CH DE FR GB IT LI LU NL SE**

1. Composé pharmaceutiquement actif de formule:

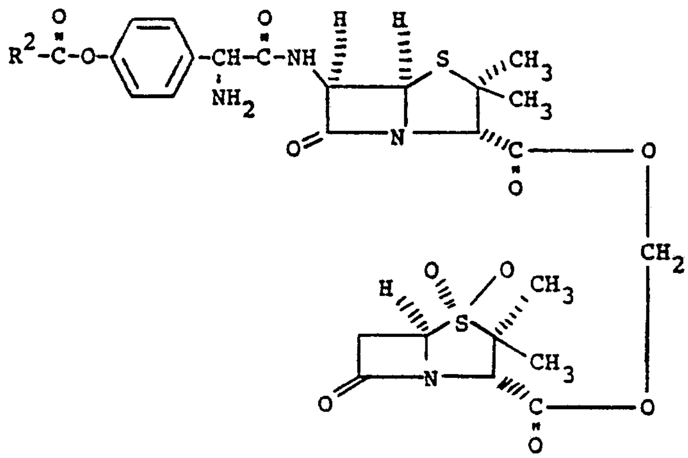

---(II)

et sels d'addition pharmaceutiquement acceptables d'un tel composé, dans lequel $R^2$ est un groupe alkyle ayant de 1 à 6 atomes de carbone.

2. Composé selon la revendication 1, caractérisé en ce que $R^2$ est un groupe isopropyle.

3. Composition pharmaceutique, convenant au traitement d'une infection bactérienne chez un mammifère, caractérisée en ce qu'elle renferme une quantité efficace sur le plan anti-bactérien d'un composé selon la revendication 1 et d'un véhicule pharmaceutiquement acceptable.

4. Procédé pour la préparation d'un composé selon la revendication 1, caractérisé en ce qu'il consiste à enlever le grope X amino-proctecteur d'un composé de formule:

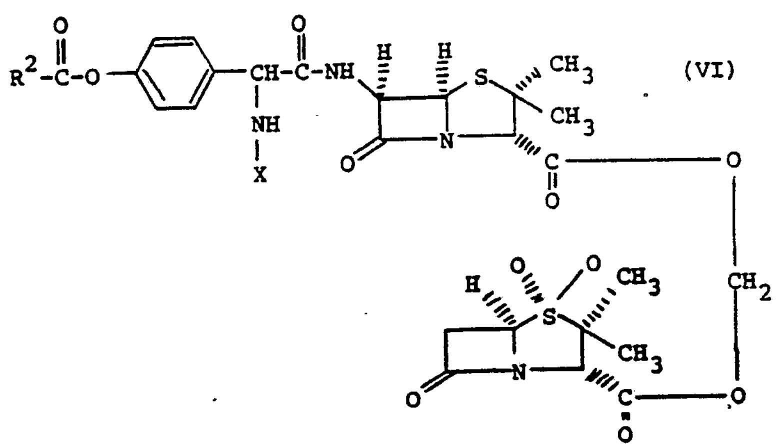

(VI)

5. Procédé selon la revendication 4, caractérisé en ce que X est un groupe 1-méthyl-2-alcoxy-carbonylvinyle ayant de 1 à 3 atomes de carbone dans ledit motif alcoxy, un groupe benzyloxycarbonyle ou un groupe 4-nitro-benzyloxycarbonyle.

**Revendications pour l'Etat Contractant: AT**

1. Procédé de préparation d'un composé pharmaceutiquement actif de formule:

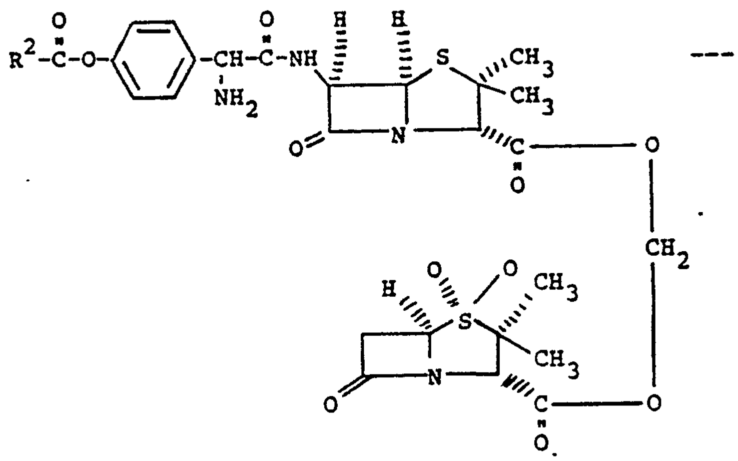

---(II)

ou d'un sel d'addition acide pharmaceutiquement acceptable d'un tel composé dans lequel $R^2$ est un groupe alkyle ayant de 1 à 6 atomes de carbone; caractérisé en ce qu'il consiste à enlever le groupe X amino-protecteur d'un composé de formule:

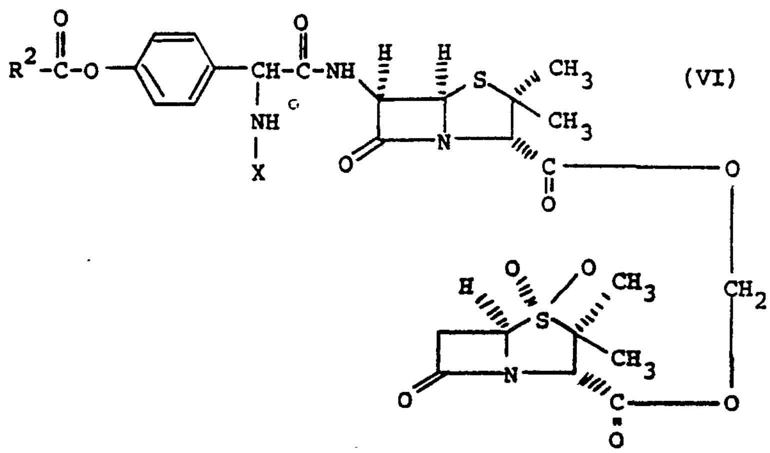

(VI)

2. Procédé selon la revendication 1, caractérisé en ce que $R^2$ est un groupe isopropyle.

3. Procédé selon la revendication 1 ou 2, caractérisé en ce que X est un radical 1-méthyl-2-alcoxy-carbonylvinyle (ayant de 1 à 3 atomes de carbone dans ledit motif alcoxy), un groupe benzyloxycarbonyle ou un groupe 4-nitro-benzyloxycarbonyle.

4. Procédé selon la revendication 3, caractérisé en ce que X est ledit groupe 1-méthyl-2-alcoxycarbonyl-vinyle et en ce qu'il est enlevé par exposition du composé de formule VII à un système de solvant aqueux ou partiellement aqueux à pH acide.